# EUROPÄISCHE PATENTSCHRIFT

(11) **EP 0 882 466 B1**
(45) Veröffentlichungstag und Bekanntmachung des Hinweises auf die Patenterteilung: **07.09.2005**
(21) Anmeldenummer: 98810494.9
(22) Anmeldetag: 28.05.1998
(51) Int. Cl.: A61M 5/14, A61M 5/168, A61M 39/24

(54) **Vorrichtung zur dosierten Verabreichung einer Medikamentflüssigkeit**
Device for the dosed application of a liquid drug
Dispositif pour l'application dosée d'un médicament liquide

(30) Priorität: 05.06.1997 DE 19723648
(43) Veröffentlichungstag der Anmeldung: 09.12.1998
(73) Patentinhaber: Disetronic Licensing AG, 3401 Burgdorf (CH)
(72) Erfinder: Kindler, Beat, 3415 Hasle-Rüegsau (CH); Peter, Daniel, 3172 Niederwangen (CH); Haueter, Ueli, 3506 Grosshöchstetten (CH); Aeschlimann, Reto, 3426 Aefligen (CH)
(74) Vertreter: Schwabe - Sandmair - Marx

(56) Entgegenhaltungen:
- WO-A-95/16480
- WO-A-97/02059
- WO-A-98/05378
- DE-A- 19 633 530
- US-A- 4 615 693
- US-A- 4 681 132
- US-A- 4 919 167

## Beschreibung

Die Erfindung betrifft eine Vorrichtung zur dosierten Verabreichung einer Medikamentflüssigkeit nach dem Oberbegriff von Anspruch 1.

Bekannte Infusionssysteme lagern das zu verabreichende Medikament in einem Behältnis, üblicherweise einer Ampulle, in der sich eine Trägerflüssigkeit mit dem darin gelösten Medikament - im folgenden einfach Medikamentflüssigkeit genannt - zwischen einem beweglichen Stopfen und einem Behältnisauslass befindet. Am Behältnisauslass ist ein Katheter mit seinem hinteren Ende angeschlossen. Am vorderen Katheterende sitzt eine Nadel, die zum Verabreichen der Medikamentflüssigkeit in den menschlichen oder tierischen Körper eingeführt wird und dort meist über eine oft mehrtägige Dauer der Verabreichung verbleibt. Befindet sich dabei das Behältnis mit der Medikamentflüssigkeit in einer größeren Höhe als das vordere Katheterende bzw. die Nadel, so besteht bei ausreichender Höhendifferenz zwischen dem Behältnis und dem vorderen Katheterende die Gefahr, dass sich das Behältnis durch die Kraft der Flüssigkeitssäule allmählich selbst entleert.

In der Insulintherapie mit tragbaren Infusionsgeräten, beispielsweise Pumpengeräten, können die verwendeten Katheter Längen von mehr als 1 m aufweisen. So hat der bislang längste mit diesem Infusionsgerät verwendete Katheter eine Länge von immerhin 1,1 m. Wird das Gerät mit dem Behältnis vertikal über dem Verwender angeordnet, beispielsweise nachts, so entsteht ein hydrostatischer Bodendruck von etwa 0.1 bar, falls neben dem rein statischen Druck aufgrund des Eigengewichts der Medikamentflüssigkeit keine weiteren Effekte, beispielsweise Reibungsverluste, Auslaufeffekte oder Kapilarwirkungen, berücksichtigt werden und für die Medikamentflüssigkeit die Dichte von Wasser angenommen wird.

Zum Verhindern des unerwünschten Auslaufens aufgrund des Drucks der Flüssigkeitssäule könnte die Wandreibung zwischen dem im Behältnis frei gleitend aufgenommenen Stopfen und der Behältniswandung vergrößert werden, was jedoch anderweitige Nachteile mit sich bringen würde. Eine andere Lösung wäre, den Stopfen am Abtriebsglied zu fixieren, so dass der Stopfen ein Absinken der Flüssigkeitsoberfläche im Behältnis verhindert und dadurch einer Selbstentleerung vorbeugt. Bekannte Systeme verschrauben den Stopfen mit dem Abtriebsglied. Dies treibt jedoch die Kosten des Geräts. Ferner ist diese Lösung bei vorkonfektionierten Ampullen nicht anwendbar, da der Stopfen für eine Schraubverbindung nicht vorbereitet ist.

Die internationale Anmeldung WO 97/02059 betrifft eine Infusionspumpe mit einem Pumpengehäuse und einem Sicherheitsventil, das alleine durch Schwerkraft verursachte Medikamentenausschüttung verhindern soll. Das Anschlussgehäuse der Pumpe ist an dem Pumpengehäuse lösbar befestigt. Ferner ist es mit seinem stromaufwärtigen Ende über einen Kathether und einen Einlasskonnektor mit einem sackförmigen Medikamentenreservoir verbunden.

Die Internationale Patentanmeldung WO 95/16480 offenbart eine Infusionsvorrichtung mit einem Medikamentenbehältnis, einem davon abgehenden Kathether, einer an dem Kathether angeordneten Klemme, einer an den Kathether angeschlossene Pumpe, einem von der Pumpe zum Patienten führenden weiteren Kathether und einen in dem weiteren Kathether angeordnenten Sicherheitsventil. Mittels der Klemme für den ersten Kathether und dem Sicherheitsventil soll eine unerwünschte Förderung des Medikamentenfluids aufgrund der Schwerkraft verhindert werden.

Die Erfindung hat es sich zur Aufgabe gemacht, bei einer Vorrichtung zur dosierten Verabreichung einer Medikamentflüssigkeit aus einem Flüssigkeitsbehältnis ein unkontrolliertes Auslaufen unter den im täglichen Gebrauch herrschenden Verhältnissen zu verhindern.

Diese Aufgabe wird durch den Gegenstand von Anspruch 1 gelöst.

Die Erfindung geht von einer Vorrichtung zur dosierten Verabreichung einer Medikamentflüssigkeit aus, bei der die Medikamentflüssigkeit in einem Behältnis enthalten ist, aus dem sie durch Vorschub eines im Behältnis beweglich aufgenommenen Stopfens auf einen Behältnisauslass zu in dosierter Weise zu ihrer Verabreichung verdrängt wird. Unmittelbar am Auslass des Behältnisses, wo ein Auslassstück bzw. -stutzen zum Anschließen eines Katheters vorhanden ist, ist solch ein Katheter mit seinem hinteren Ende angeschlossen. Üblicherweise handelt es sich um einen schlauchförmigen Katheter. Die Verwendung eines starren Katheters wäre jedoch ebenso möglich. Das vordere, freie Ende des Katheters ist mit einer Nadel zur Verabreichung des Medikaments verbunden oder mit solch einer Nadel verbindbar. Unter einer Verabreichung wird sowohl eine Infusion als auch eine Injektion und auch eine Kombination beider Verabreichungsarten verstanden. Insbesondere zielt die Erfindung auf die Verwendung bei Infusionsvorrichtungen bzw. -geräten ab. Besonders bevorzugt handelt es sich um tragbare Pumpengeräte in der Insulinbehandlung.

Erfindungsgemäß ist zwischen dem Behältnisauslass und der Nadel zum Verabreichen des Medikaments ein Ventil in einem Strömungsquerschnitt der Medikamentflüssigkeit angeordnet. Das Ventil ist so dimensioniert, dass es zum Verhindern einer Selbstentleerung einen Durchfluss auf das vordere Ende des Katheters zu nur zulässt, wenn der in diese Richtung wirkende Flüssigkeitsdruck größer ist als ein auf dem Ventil lastender Druck infolge des Eigengewichts der Flüssigkeitssäule in der Vorrichtung. Handelt es sich um eine in Serie gefertigte Vorrichtung, für die ein ganzes Sortiment von Kathetern mit unterschiedlichen Längen zur Verfügung steht, so ist das Ventil für den Einsatz des längsten Katheters, d.h. für den Fall der maximal möglichen Flüssigkeitssäule, dimensioniert.

Die Erfindungsgemäße Vorrichtung zur dosierten Verabreichung einer Medikamentenflüssigkeit ist dadurch gekennzeichnet, dass die Medikamentenflüssigkeit durch Vorschub eines Stopfens durch den Auslass verdrängt wird und dass das Anschlussgehäuse lösbar an dem Auslass angeschlossen ist und eine Verbindungsnadel so lagert, dass die Verbindungsnadel eine den Auslass verschließende Membran durchsticht, wenn das Anschlussgehäuse angeschossen wird.

Das Ventil ist vorteilhaft als Einwegventil ausgeführt, das einen Rückfluss in das Behältnis idealerweise verhindert. Besonders bevorzugt handelt es sich um ein Rückschlagventil.

Um die dosierte Medikamentverabreichung möglichst wenig zu behindern, aber dennoch ein Auslaufen sicher zu unterbinden, ist das Ventil vorzugsweise so ausgelegt, dass es den Durchfluss in Richtung auf das vordere Ende des Katheters erst zulässt, wenn der Flüssigkeitsdruck in diese Richtung den maximal möglichen Druck der Flüssigkeitssäule, vorzugsweise multipliziert mit einem Sicherheitsfaktor, übersteigt. Da es sich im vorliegenden Fall um eine Anwendung des Ventils im medizinaltechnischen Bereich handelt, entspricht dieser Sicherheitsfaktor besonders bevorzugt dem Wert 3. Bei einer maximalen Katheterlänge von etwa 1 m und vernachlässigbarer Flüssigkeitssäule im Behältnis beträgt der maximale Flüssigkeitsdruck am freien Ende des Katheters etwa 0.1 bar, so dass das Ventil in diesem Falle so ausgelegt wird, dass es erst öffnet, wenn der Flüssigkeitsdruck 0.3 bar übersteigt. Dies ist auch der Dimensionierungsfall für die bevorzugte Verwendung in einer tragbaren Infusionspumpe.

Obgleich das Ventil grundsätzlich an beliebiger Stelle zwischen dem Behältnisauslass und der Verabreichungsnadel sitzen könnte, wird es bevorzugterweise so nah als möglich beim Auslass des Behältnisses angeordnet. In dieser Anordnung verhindert das Ventil, sofern es als Rückschlagventil ausgelegt ist, auch noch ein unerwünschtes Rückfließen in das Behältnis am wirkungsvollsten.

Zur Aufnahme des Ventils ist beispielsweise ein Auslassstutzen im Bereich des Behältnisauslasses geeignet.

Nach einem besonders bevorzugten Ausführungsbeispiel ist das Ventil in einem als Anschlussstück für den Katheter dienenden Gehäuse angeordnet. Das Ventil ist dadurch zusammen mit dem Katheter leicht auswechselbar.

Das Ventil umfasst als Dichtelement einen Ventilkörper, vorzugsweise aus elastischem Material, der im montierten Zustand eine Zuführleitung, d.h. deren wenigstens eine Öffnung, verschließt. Die Zuführleitung unmittelbar vor dem Ventilkörper kann durch eine Verbindungsnadel gebildet werden, die beim Anschließen des Katheters an den Behältnisauslass eine Membran durchsticht und so die Flüssigkeitsverbindung herstellt. Der letzte Abschnitt der Zuführleitung mit der durch den Ventilkörper verschlossenen Öffnung kann auch durch das genannte Gehäuse gebildet werden, in dem solch eine Verbindungsnadel beispielsweise aufgenommen ist.

Der Verschluss des Strömungsquerschnitts kann durch die Wirkung einer Dichtlippe mit schmaler, genau definierter Kontaktfläche erzielt werden, die am Ventilkörper oder an der Zuführleitung ausgebildet ist. Die Erzielung der Dichtwirkung mittels einer die wenigstens eine Öffnung der Zuführleitung umschließenden Dichtlippe hat den Vorteil, dass der Druck, bei dem das Ventil öffnet und schließt, definiert werden kann. Die Ausbildung an der Zuführleitung hat den Vorteil der einfachen Ventilkörperfertigung.

In einer anderen Ausführungsform ist der elastische Ventilkörper hohlzylindrisch und wird wie ein Schlauch auf die Zuführleitung aufgezogen. Die wenigstens eine zu verschließende

Öffnung der Zuführleitung ist in einer Mantelfläche der Zuführleitung angeordnet. Die Zuführleitung und der darüber gestülpte Ventilkörper wirken zusammen in der Art eines Fahrradschlauchventils.

Als konstruktiv besonders einfacher Ventilkörper kommt ein Dichtpfropfen aus elastischem Material zum Einsatz, der den Strömungsquerschnitt der Medikamentflüssigkeit pfropfenartig verschließt. Der Dichtpfropfen kann mit einer vorgefertigten Durchlassöffnung versehen sein, die im eingesetzten Zustand jedoch zumindest solange verschlossen ist, bis der genannte, ausreichend hohe Flüssigkeitsdruck auf dem Dichtpfropfen lastet. Um die Fertigung des Ventils zu vereinfachen, wird der Durchlass erst nach dem Einsetzen des Dichtpfropfens in das Gehäuse geschaffen, indem die Verbindungsnadel bei ihrem Einsetzen in das Gehäuse zunächst den Dichtpfropfen vollkommen durchsticht und anschließend wieder ein kleines Stück zurückgezogen wird, so dass der so geschaffene Durchlass durch die elastische Masse des Dichtpfropfens wieder verschlossen wird.

Weitere erfindungsgemäß ausgeführte Ventile weisen einen elastischen Ventilkörper auf, der in der Art einer Herzklappe wirkt.

Schließlich kann auch ein durch eine Druckfeder beaufschlagter Ventilkörper zum Einsatz kommen.

Bevorzugte Ausführungsbeispiele der Erfindung werden nachfolgend anhand von Zeichnungen erläutert. Es zeigen:
- Figur 1: eine erfindungsgemäße Vorrichtung mit einem Ventil zum Verhindern einer unkontrollierten Medikamentabgabe und
- Figuren 2-23: alternative Ausführungsbeispiele für das Ventil und dessen Anordnung nach Figur 1.

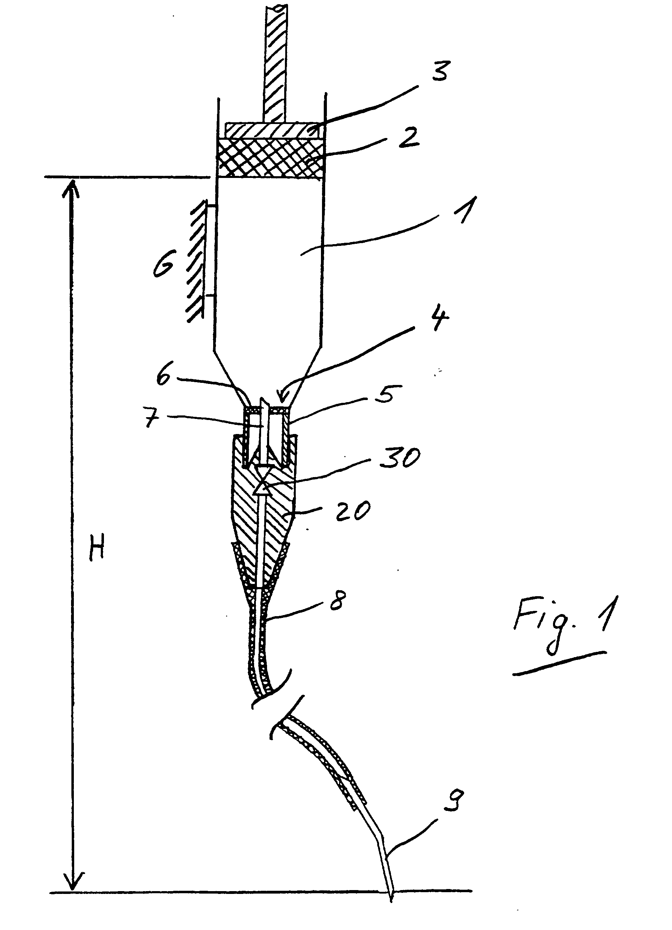

Figur 1 zeigt eine Vorrichtung zur dosierten Verabreichung einer Medikamentflüssigkeit. In der dargestellten Ausbildung kann die Vorrichtung ein Infusions- oder auch ein Injektionssystem sein. Im folgenden ist einer bevorzugten Verwendung wegen jedoch lediglich noch von Infusion, insbesondere von Insulininfusion, die Rede.

Das in seiner Trägerflüssigkeit gelöste Insulin ist in einer Ampulle 1 enthalten, die an einem Gestell oder in einem Gehäuse G befestigt ist. In der Ampulle 1 ist ein Stopfen 2 frei beweglich gleitgeführt aufgenommen. Durch Vorschub des Stopfens 2 in Richtung auf einen Auslass 4 des Behältnisses 1 zu wird die Insulinflüssigkeit aus der Ampulle 1 verdrängt. Ein Abtriebsglied 3 einer vorzugsweise motorischen Antriebsvorrichtung für den Stopfen 2 bewirkt durch Andruck auf die Stopfenrückseite dessen Vorschub. Eine form- oder stoffschlüssige Verbindung zwischen dem Abtriebsglied 3 und dem Stopfen 2 besteht nicht. Der Stopfen 2 wird in der Ampulle 1 lediglich durch die zur Erzielung der Dichtheit unerlässlichen Wandreibungskräfte gehalten. Die Vorschubbewegung des Abtriebsglieds 3 und damit des Stopfens 2 wird exakt gesteuert, um das Insulin in fein dosierter Weise durch den Auslass 4 abzugeben.

Der Auslass 4 ist vor der ersten Verwendung der Ampulle 1 durch eine Membran 6 flüssigkeitsdicht verschlossen. Beim Anschließen eines Infusionskatheters 8 wird die Membran 6 von einer Verbindungsnadel 7 durchstochen, die in einem Gehäuse 20 sitzt. Das Gehäuse 20 dient als Anschlussstück für den Katheter 8. Dessen hinteres Ende ist auf das sich verjüngende vordere Ende des Gehäuses bzw. Anschlussstücks 20 aufgezogen. Mit seinem hinteren Ende ist das Anschlussstück 20 auf einen den Auslass 4 verlängernden Auslassstutzen 5 auf- oder eingeschraubt oder mittels Klickverschluss daran befestigt. Am vorderen, freien Ende des Katheters 8 ist eine Infusionsnadel 9 befestigt.

In dieser Anordnung, bei der sich ein Patient die Infusionsnadel 9 unter die Haut gestochen und sein Pumpgerät mit der Ampulle 1 um die Höhendifferenz H über der Einstichstelle befestigt oder abgelegt hat, beispielsweise nachts, lastet auf dem vorderen Ende der Injektionsnadel 9 ständig der durch die Höhendifferenz H bewirkte Druck der Flüssigkeitssäule zwischen dem vorderen Ende der Infusionsnadel 9 und der Flüssigkeitsoberfläche in der Ampulle 1. Infolge dieses Drucks würde permanent Insulin am vorderen Ende der Infusionsnadel 9 freigesetzt werden.

Um dies zu verhindern ist im Anschlussstück 20 ein passives Ein- oder Zweiwegventil 30, insbesondere ein Rückschlagventil, im Strömungsquerschnitt der Insulinflüssigkeit angeordnet. Das Ventil 30 lässt einen Durchfluss vom Auslass 4 in den Katheter 8 überhaupt erst zu, wenn der in die Richtung der Infusionsnadel gerichtete Flüssigkeitsdruck um einen vorgegebenen Sicherheitsfaktor größer ist, als der Druck der Flüssigkeitssäule mit der maximalen Höhendifferenz H. Wirkt das Ventil 30 als passives Einwegventil, beispielsweise als einfaches Rückschlagventil, verhindert es zudem einen Rückfluss der Medikamentflüssigkeit in die Ampulle 1, wodurch die Funktionssicherheit der Pumpe zusätzlich erhöht wird.

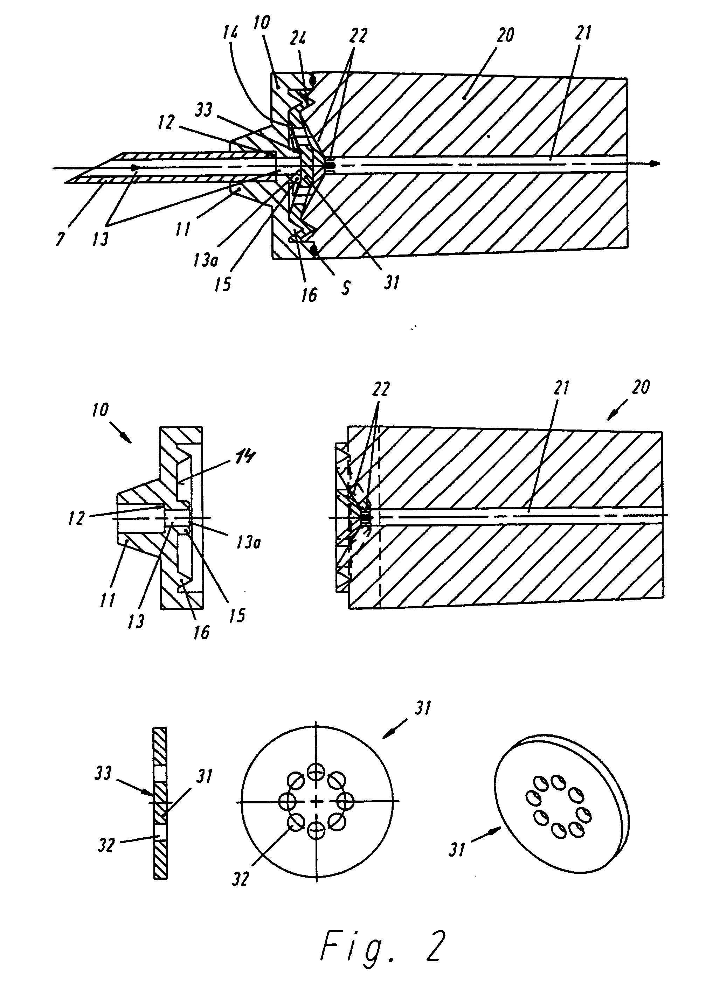

Figur 2 zeigt ein erstes Ausführungsbeispiel für ein Anschlussstück mit Ventil. Das Anschlussstück ist in diesem Ausführungsbeispiel zweiteilig aus einer stromaufwärtigen ersten Gehäushälfte 10 und einer stromabwärtigen zweiten Gehäushälfte 20 zusammengesetzt. Das gesamte Gehäuse 10, 20 ist rotationssymmetrisch zu seiner in Strömungsrichtung verlaufenden, geraden Mittellängslinie. Eine Trennfläche zwischen den beiden Gehäusehälften 10 und 20 steht senkrecht zur Strömungsrichtung. In die Trennfläche ist ein Ventilkörper 31 aus einem elastischen Material eingelegt und durch Zusammenfügen der beiden Gehäusehälften 10 und 20 umlaufend entlang seines äußeren Umfangsrandes zwischen den beiden Gehäusehälften 10 und 20 verpresst. Der Ventilkörper 31 dichtet so die Trennfläche ab. Desweiteren sind die beiden Gehäushälften 10 und 20 ringförmig umlaufend bei S miteinander verschweißt. Falls werkstofftechnisch möglich, wird auch der Ventilkörper 31 vorzugsweise in diese Schweißverbindung mit einbezogen.

Der Ventilkörper 31 wird durch eine Membran-Kreisscheibe, gegebenenfalls mit axial vorstehendem Ringsteg, gebildet. Im eingebauten Zustand dient ihr zentraler Bereich als Ventilteller. Den kreisförmigen Ventilteller umgeben eine oder mehrere Durchgangsöffnungen 32. Der äußere Kreisringbereich dient der bereits genannten Befestigung zwischen den beiden Gehäusehälften 10 und 20.

Beide Gehäusehälften 10 und 20 sind mit je einer zentralen Durchgangsbohrung 13 und 21 versehen. Die beiden Bohrungen 13 und 21 fluchten. In die Durchgangsbohrung der ersten Gehäusehälfte 10 ist in einem stromaufwärtigen Gehäusekonus 11 die Verbindungsnadel 7 eingelegt und befestigt. Um eine verwirbelungsfreie Strömung zu erzielen, weist die Bohrung in der ersten Gehäusehälfte 10 in ihrem stromaufwärtigen Bereich einen dem Außendurchmesser und in ihrem stromabwärtigen, daran anschließenden Bereich einen dem Innendurchmesser der Nadel 7 entsprechenden Durchmesser auf. Es wird hierdurch nicht nur eine Zuführleitung 13 mit durchgehend glatter Wandung, sondern auch ein Schulteransatz 12 geschaffen, an den die Nadel 7 mit ihrem stromabwärtigen Ende anstößt. Am stromabwärtigen Ende der Zuführleitung 13 bildet die erste Gehäusehälfte 10 einen die Leitungsöffnung 13a umschließenden, als Dichtlippe 15 wirkenden Steg, dessen Stirnfläche vorzugsweise gerundet ist.

Der Ventilkörper 31 ist über die ringförmige Dichtlippe 15 vorgespannt. Hierzu fällt die erste Gehäusehälfte 10 in ihrem Inneren von der in Strömungsrichtung vorstehenden Dichtlippe 15 entgegen der Strömungsrichtung ab. Die Dichtlippe 15 wird so von einer zurückgenommenen Kreisringfläche 14 umgeben. Um die Kreisringfläche 14 läuft ein, wie bereits die Dichtlippe 15, in Strömungsrichtung von der Kreisringfläche 14 erhaben vorstehender Ringwulst 16 um, dem auf der Seite der zweiten Gehäusehälfte 20 eine entsprechende Ausnehmung gegenüberliegt. Vor dem Zusammensetzen der beiden Gehäusehälften 10 und 20 liegt der Ventilkörper 31 von der Dichtlippe 15 geringfügig beabstandet in ihrem äußeren Umfangsbereich auf der ersten Gehäusehälfte 10 auf und ist dabei auch ein Stück von der Kreisringfläche 14 beabstandet. Durch Zusammendrücken der beiden Gehäusehälften 10 und 20 wird die Ventilkörperscheibe 31 über der Dichtlippe 15 zum äußeren Umfangsrand der Kreisringfläche 14 hin gebogen und dadurch gleichzeitig auf die Dichtlippe 15 vorgespannt. Der Ringwulst 16 drückt die Ventilkörperscheibe 31 in die Ausnehmung der zweiten Gehäusehälfte 20, wodurch eine ringförmige Klemmung der Ventilkörperscheibe und gleichzeitig eine gute Abdichtung erzielt werden. Die Dichtlippe 15 und der Ventilkörper 31 dichten den Strömungsquerschnitt entlang der umlaufenden, schmalen Kontaktfläche 33 ab, mit der die Dichtlippe 15 gegen den Ventilkörper 31 drückt.

Durch die Ausbildung des Ventilkörpers 31 als einfache Membranscheibe und Anordnung der Dichtlippe 15 am vergleichsweise starren Gehäuse werden besonders gut reproduzierbare Ventileigenschaften sowie eine einfache Herstellung des elastischen Ventilkörpers 31 erreicht. Die stromabwärtige Stirnseite der ersten Gehäusehälfte 10, über der der Ventilkörper 31 gespannt ist und dieser Ventilkörper 31 selbst sind so dimensioniert, dass der Ventilkörper 31 in der in Figur 2 im montierten Zustand dargestellten Lage mit solch einer Vorspannkraft gegen die Kontaktfläche 33 mit der Dichtlippe 15 gepresst wird, dass der Anpressdruck in der Kontaktfläche 33 größer ist als der Druck der in der Vorrichtung nach Figur 1 durch die auf dem Strömungsquerschnitt an der Dichtlippe lastende Flüssigkeitssäule bei der größtmöglichen Höhendifferenz H entsteht.

Wird dieser maximale Druck der Flüssigkeitssäule um einen vorgegebenen Sicherheitsfaktor überschritten, so hebt der Ventilkörper 31 von seinem Sitz an der Dichtlippe 15 ab. Nun kann die Insulinflüssigkeit durch die Zuführleitung 13 hindurchströmen, die Dichtlippe 15 umströmen, durch die eine oder mehrere konzentrisch um die Dichtlippe 15 angeordneten Durchlassöffnungen 32 des Ventilkörpers 31 hindurchtreten und stromabwärts vom Ventilkörper 31 über die als Auslassleitung 21 dienende Bohrung in den Schlauchkatheter abfließen. In die andere Richtung wirkt das Ventil als sichere Rücklaufsperre.

Unmittelbar hinter dem Ventilkörper 31 ist in der zweiten Gehäusehälfte 20 ein Hohlraum ausgenommen, in den sich der Ventilkörper 31 hinein ausdehnen kann. Um zu verhindern, dass der Ventilkörper 31 mit seiner stromabwärtigen Rückseite an der Innenwandung der zweiten Gehäusehälfte 20 zum Anliegen kommt mit der Gefahr, dass die Durchflussöffnungen 32 verstopft werden, ragen von der inneren Wandung der zweiten Gehäusehälfte 20 in Richtung auf den Ventilkörper 31 zu radial verlaufende Abstandsrippen 22 ab.

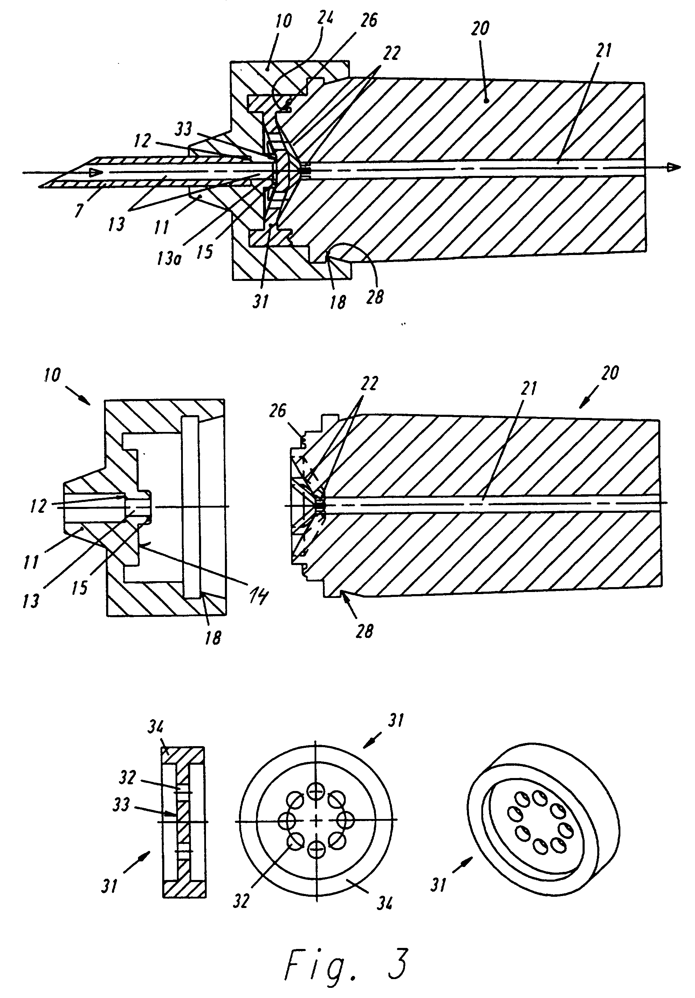

Figur 3 zeigt eine vom Ventil nach Figur 2 abgewandelte Ausführungsform. Hinsichtlich der Funktion des Ventils bestehen keine Unterschiede. Der elastische Ventilkörper 31 wird in diesem Ausführungsbeispiel durch eine im Querschnitt Doppel-T-förmige Kreismembran gebildet. Der beidseitig abragende Ringsteg 34 am äußeren Umfangsrand der Membranscheibe dient der Befestigung an der Fügestelle der beiden Gehäusehälften 10 und 20. Gleichzeitig stellt er eine vergleichsweise große Verformungsmasse für das Abdichten des Gehäuses zur Verfügung. In diesem Ausführungsbeispiel werden die beiden Gehäusehälften 10 und 20 durch eine Schnappverbindung miteinander verbunden. Zum Herstellen der Schnappverbindung wird die zweite Gehäusehälfte 20 in die stromabseitig sich öffnende, hohlzylindrische erste Gehäusehälfte 10 eingeschoben und verrastet. Hierzu ist die zweite Gehäusehälfte 20 mit einer an ihrem äußeren Umfang umlaufenden Nut 28 versehen, und die erste Gehäusehälfte 10 weist in ihrem hohlzylindrischen Bereich eine radial nach innen vorstehende, umlaufende Rastrippe 18 auf, mit der sie in die Nut 28 einrastet.

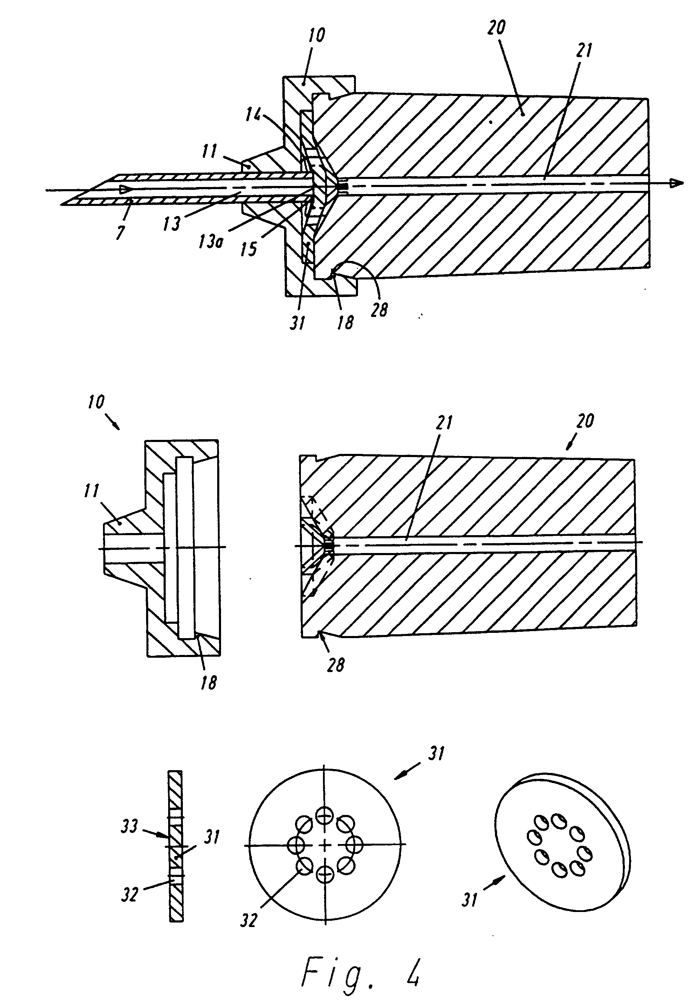

Ein drittes Ausführungsbeispiel, bei dem die Dichtlippe am Gehäuse ausgebildet ist, zeigt Figur 4. In diesem Ausführungsbeispiel wird der Ventilkörper 31 nach Figur 2 in Verbindung mit einer grundsätzlich aus Figur 3 bekannten Schnappverbindung eingesetzt. Im Ausführungsbeispiel der Figur 4 ist die erste Gehäusehälfte 10 einfacher konstruiert, indem die Aufnahme für die Verbindungsnadel 7 durch eine einfache Durchgangsbohrung gebildet wird, in die die Verbindungsnadel 7, nachdem der Ventilkörper 31 in die erste Gehäusehälfte 10 eingesetzt und die beiden Gehäusehälften 10 und 20 miteinander verschnappt wurden, bis zu einer Lage relativ zur Fläche 14 ein- und durchgeschoben wird, in der sie den danach eingelegten Ventilkörper 31 mit der erforderlichen Vorspannkraft spannt. Das stromabwärtige Ende der Verbindungsnadel 7 bildet dabei die Dichtlippe 15. Es ist für diesen Zweck abgerundet, damit der Ventilkörper 31 nicht beschädigt werden kann. Durch das nachträgliche Einsetzen der Nadel 7 können Fertigungstoleranzen des Ventilkörpers 31 ausgeglichen werden, indem die Nadel 7 sehr genau soweit eingeschoben wird, dass die gewünschte Anpresskraft des Ventilkörpers 31 an die nadelseitige Dichtlippe erzielt wird. Hinsichtlich der weiteren Detailes wird auf die Beschreibung zu den Figuren 2 und 3 verwiesen.

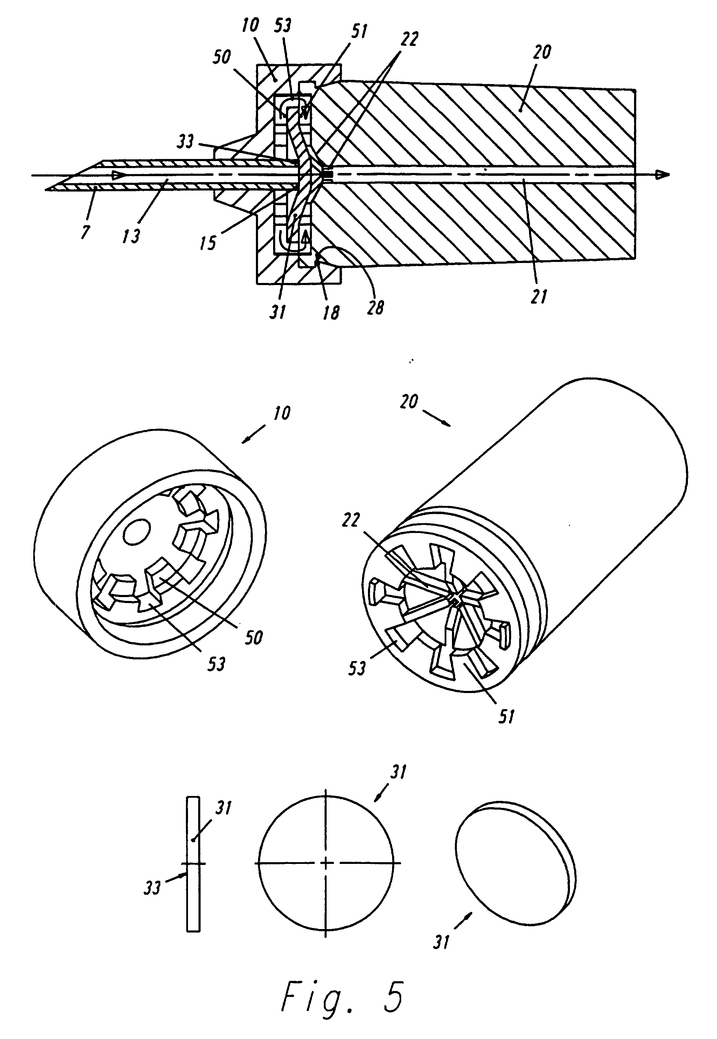

Auch Figur 5 zeigt ein Ausführungsbeispiel, bei dem die Dichtlippe 15 gehäuseseitig vorgesehen ist. Sie wird wie im Ausführungsbeispiel nach Figur 4 durch das rückwärtige Ende der Verbindungsnadel 7 gebildet. Im Unterschied zu den vorstehend beschriebenen Ventilbauweisen weist der Ventilkörper 31, welcher aus einer aus elastischem Material ausgestanzten Membran-Kreisscheibe besteht, keine Durchflussöffnungen mehr auf. Der Ventilkörper 31 wird zwischen den Gehäuseteilen 10 und 20 auch nicht mehr auf einem Kreisring eingespannt, sondern nur noch auf einigen Kreisringsegmenten 50 und 51, wodurch die Medikamentflüssigkeit durch Durchlasskanäle 53 ausserhalb des Kreisscheiben-Durchmessers des Ventilkörpers 31 abgeleitet und zur Auslassbohrung 21 geführt wird.

Die Vorspannung des Ventilkörpers 31 erfolgt auch hier wieder, nachdem der Ventilkörper 31 in den einen Gehäuseteil eingelegt und die beiden Gehäuseteile zusammengebaut wurden, durch Einschieben der Verbindungsnadel 7 in den stromaufwärtigen Teil des Gehäuses 10, bis der Ventilkörper 31 so stark vorgespannt ist, dass der gewünschte Durchbruchdruck für das Ventil erreicht ist.

Übersteigt der Flüssigkeitsdruck in der Zuführleitung 13 den Anpressdruck des Ventilkörpers 31 an die Dichtlippe 15, so wird an der Kontaktfläche 33 ein ringförmiger Durchströmspalt freigegeben. Die Insulinflüssigkeit kann durch diesen Ringspalt und anschließend durch die Durchlasskanäle 53 den Ventilkörper 31 umströmen und über die Auslassleitung 21 abfließen.

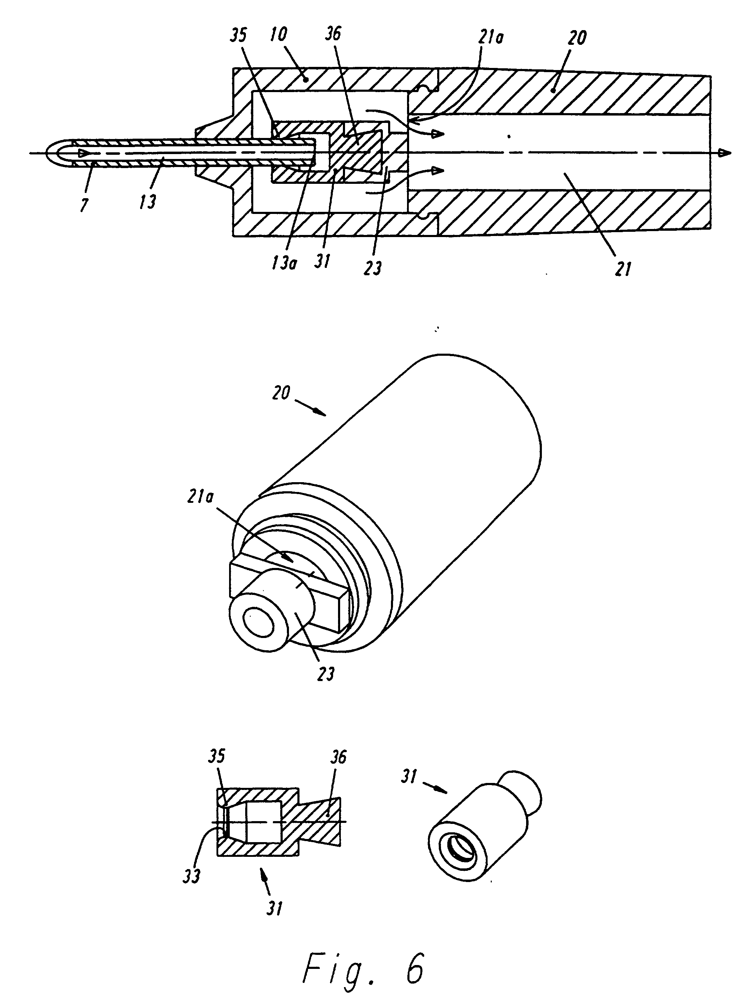

In den Figuren 6 bis 9 sind weitere Ventilausführungen dargestellt, bei denen die gewünschte Dicht- bzw. Durchschlagcharakteristik mittels Dichtlippe erzielt wird. Hierbei ist die Dichtlippe jedoch an dem elastischen Ventilkörper 31 vorgesehen. Auch zu diesen Beispielen sei auf die vorstehende Beschreibung hingewiesen. Es werden lediglich die charakteristischen Unterschiede dargestellt.

Beim Ventil nach Figur 6 ragt die Verbindungsnadel 7 wieder in die erste Gehäusehälfte 10 hinein. Der stromabwärtigen Öffnung 13a der Verbindungsnadel 7 gegenüberliegend ist an der zweiten Gehäusehälfte 20 der elastische Ventilkörper 31 mittels eines in seinem Längsschnitt schwalbenschwanzförmigen stromabwärtigen Ventilkörperfortsatzes 36 festgelegt. Der sich stromabwärts verbreiternde Fortsatz 36 ist in einem von der zweiten Gehäusehälfte 20 in Richtung auf die Verbindungsnadel 7 zu ragenden flanschartigen Halteteil 23 gelagert. Der Ventilkörper 31 hat die Form eines sich gegen die Strömungsrichtung öffnenden Topfs. Am stromaufwärtigen Topfrand ragt eine umlaufende Dichtlippe 35 radial nach innen vor. Im eingebauten Zustand umschließt die Dichtlippe 35 den Außenmantel der Verbindungsnadel 7 dichtend. Erst wenn der Flüssigkeitsdruck im Topfinneren den durch die Vorspannkraft auf die an der Mantelfläche der Verbindungsnadel 7 gebildete Kontaktfläche 33 ausgeübten Druck übersteigt, wird entlang dieser Kontaktfläche ein Spalt freigegeben, über den die Flüssigkeit aus der Zuführleitung 13 in den Hohlraum innerhalb des Gehäuses 10, 20 um den topfförmigen Ventilkörper 31 herum dringen kann. Der Gehäusehohlraum steht mit der Auslassleitung 21 in der zweiten Gehäusehälfte 20 über eine oder mehrere, im Ausführungsbeispiel zwei, Durchlassöffnungen 21a in Verbindung, die das Ventilkörperhalteteil 23 in seinem Fußbereich freilässt.

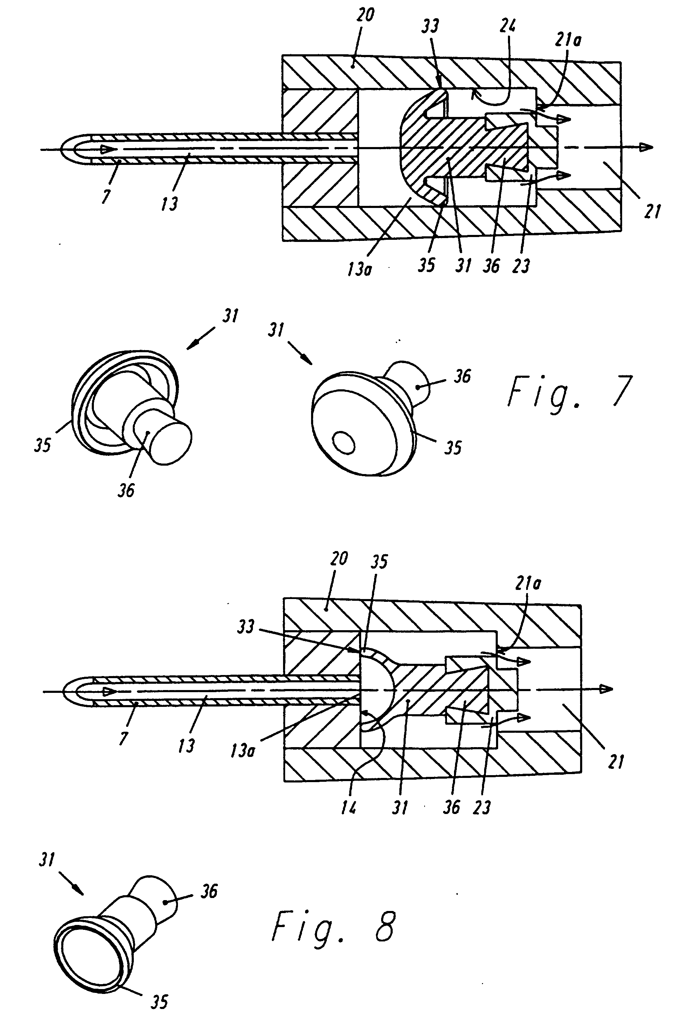

Im Ausführungsbeispiel nach Figur 7 ist das Gehäuse besonders einfach ausgebildet. Die zweite Gehäusehälfte 20 ist hohlzylindrisch mit einer stromaufwärtigen großen Öffnung und der sich daran zentrisch anschließenden Abflussbohrung 21. In der Öffnung ist der Ventilkörper 31 aufgenommen. Die erste Gehäusehälfte 10 ist ein kreisringzylindrisches Montageeinsatzstück, in dem die Verbindungsnadel 7 fest sitzt und das seinerseits in die Öffnungs der zweiten Gehäusehälfte 20 eingeschraubt oder andersartig geeignet befestigt ist.

Der Ventilkörper 31 ist ähnlich wie der in Figur 6 an einem Halteteil 23 festgelegt. Er besitzt die Form eines Pilzes. Die gewölbte Pilzoberfläche weist gegen die Strömungsrichtung. An ihrem stromabwärtigen Umfangsrand ist die gewölbte Pilzoberfläche in Ausbildung der Dichtlippe 35 gegen die Innenwandung 24 der Öffnungsbohrung im Gehäuse 20 vorgespannt.

Figur 8 zeigt ein Ausführungsbeispiel mit einem in etwa eine hohle Halbkugel bildenden Ventilkörper 31. Die Befestigung dieses Ventilkörpers 31 im Gehäuse entspricht der des Ventilkörpers nach den Figuren 6 und 7. Das Gehäuse entspricht dem Gehäuse von Figur 7. Der Ventilkörper 31 ist mit seiner stromaufwärtigen, umlaufenden, die Kontaktfläche 33 bildenden Stirnfläche gegen eine die stromabwärtige Öffnung 13a der Zuführleitung 13 umschließende, einfach plane Gegenfläche des Montageeinsatzstücks 10 gepresst. Die Zuführleitung 13 wird durch den Ventilkörper 31 glockenartig abgeschlossen.

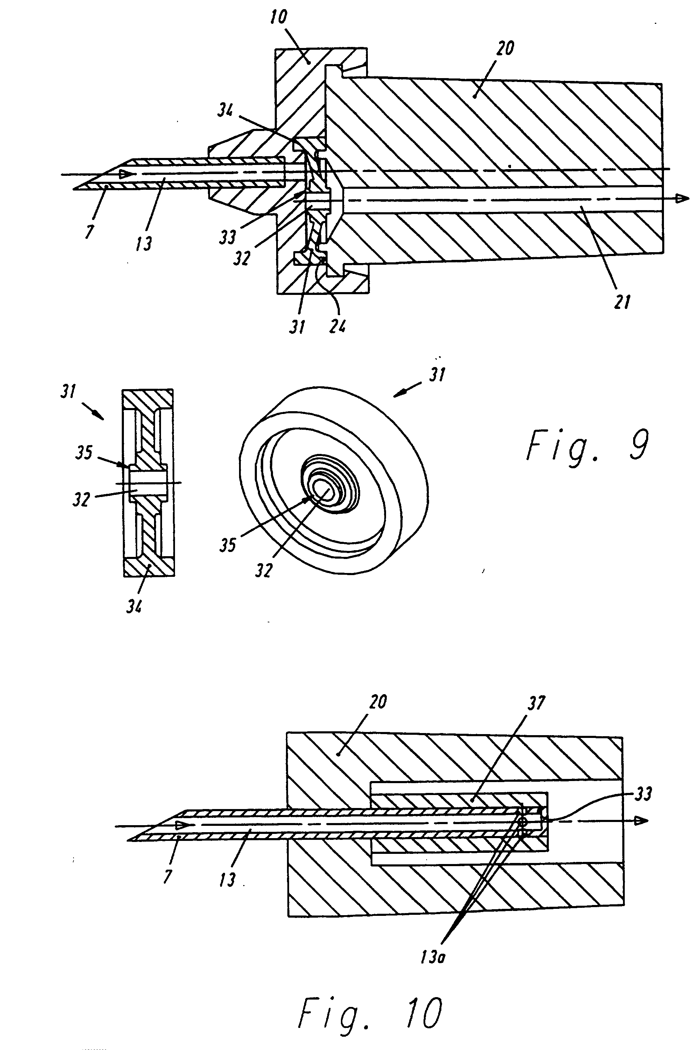

Der beim Ausführungsbeispiel nach Figur 9 verwendete Ventilkörper 31 unterscheidet sich von den vorstehend beschriebenen im wesentlichen dadurch, dass der Flüssigkeitsdruck sich nicht innerhalb der Dichlippe 35, sondern in einem Ringraum um diese Dichtlippe 35 herum aufbaut.

Beim Ventilkörper 31 nach Figur 9 wird eine zentrale Durchgangsöffnung 32, vorzugsweise eine einfache Durchgangsbohrung, von der Dichtlippe 35 umschlossen. Die Dichtlippe 35 wird von einer zurückgenommenen Ringfläche umgeben, die ihrerseits durch einen in die gleiche Richtung wie die Dichtlippe 35 von der zurückgenommenen Ringfläche abragenden Ringsteg 34 eingefasst wird. Vorzugsweise ist die Dichtlippe 35 ihrerseits dem Ringsteg 34 gegenüber ein Stück zurückgenommen. Um den Ventilkörper 31 vorspannen zu können, ist eine Ringnut an der stromabwärtigen Stirnfläche der ersten Gehäusehälfte 10 tiefer ausgenommen als es dem Abstand der Stirnflächen des äußeren Ringstegs 34 und der Dichtlippe 35 im Ausgangszustand des Ventilkörpers 31 entsprechen würde. Wird der äußere Ringsteg 34 in die Aufnahmenut der ersten Gehäusehälfte eingelegt und vollkommen in in diese Nut hineingedrückt, so wird die Dichtlippe 35 gegen die von der Ringnut umschlossene plane Fläche der ersten Gehäusehälfte 10 gepresst. Die Zuführleitung 13 in der ersten Gehäusehälfte 10 ist in diesem Ausführgunsbeispiel nicht zentral. Sie mündet an der stromabwärtigen Stirnfläche innerhalb der zurückgenommenen Ringnut an einer Stelle zwischen der Dichtlippe 35 und dem Ringsteg 34 des Ventilkörpers 31. Es wird so ein ringförmiger Druckraum gebildet. Wird der durch die elastische Vorspannung des Ventilkörpers 31 erzeugte Anpressdruck der Dichtlippe 35 in diesem Ringraum überschritten, so wird die Dichtlippe 35 von ihrer Gegenfläche abgehoben. Die Zuführleitung 13 steht dann über den Ringraum und die Durchgangsbohrung 32 mit der Auslassleitung 21 in Strömungsverbindung.

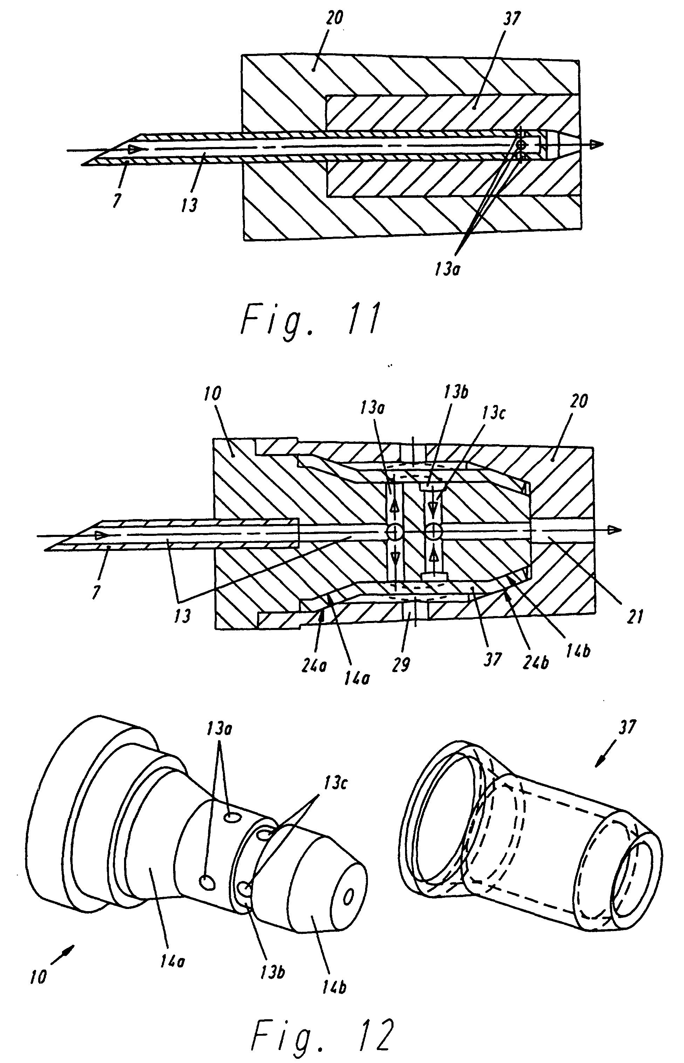

Die Figuren 10 und 11 zeigen Ventile, die in der Art eines Fahrradschlauchventils wirken. Die Ventilkörper 37 werden durch Schlauchstücke gebildet. Das Gehäuse 20 ist in beiden Ausführungsbeispielen einteilig. Der jeweilige Ventilkörper 37 kann aufgrund seiner funktionsbedingten Form auf der Nadel 7 oder im Gehäuse 20 oder zwischen beiden fest gehalten werden.

In Figur 10 ist ein einfaches Schlauchstück 37 auf das stromabwärtige Ende der Zuführleitung 13 aufgezogen. Dieses Ende wird im Ausführungsbeispiel durch die Verbindungsnadel 7 gebildet. Stirnseitig ist die Zuführleitung 13 an ihrem stromabärtigen Ende verschlossen. In ihrem in das Gehäuse 20 hineinragenden Endbereich weist die Zuführleitung 13 eine oder mehrere radiale Durchgangsöffnungen 13a auf, die vom schlauchförmigen Ventilkörper 37 umspannt und dadurch abgedichtet werden. Der stirnseitige Verschluß der Zuführleitung 13 könnte auch durch den dann sackartigen Ventilkörper 37 gebildet werden, um die Zuführleitung 13 durch Schneiden aus einer Endloskanüle herstellen zu können.

Im Ausführungsbeispiel nach Figur 11 ist der Hohlraum im Gehäuse 20, in den die Zuführleitung 13 hineinragt, mit einem elastischen Dichtmaterial 37 ausgegossen. Nach dem Gießen wird die Zuführleitung 13 durch das Dichtmaterial hindurchgestoßen. Gegebenenfalls wird das Dichtmaterial zum leichteren Einführen der Zuführleitung 13 auch vorgestochen.

In Figur 12 ist in Verbindung mit dem schlauchförmigen Ventilkörper 37 ein zweiteiliges Gehäuse 10, 20 gezeigt. Die Flüssigkeit aus der Ampulle wird über die Zuführleitung 13 über mindestens eine Verbindung 13a, vorzugsweise eine Verbindungsbohrung, an die Mantelfläche der ersten Gehäusehälfte geführt. Stromabwärts von der Verbindung 13a ist an der äußeren Mantelfläche der ersten Gehäusehälfte 10 wenigstens ein weiterer Verbindungskanal 13c, der in eine ausgenommen Nut 13b mündet. Der wenigstens eine weitere Verbindungskanal 13c führt zur Auslassleitung 21 in den Katheter. Die Verbindungsbohrung 13a und die Nut 13b sind durch einen von der Mantelfläche der ersten Gehäusehälfte gebildeten Zwischensteg voneinander getrennt. Der Ventilkörper 37 liegt eng gespannt um die Mantelfläche der ersten Gehäusehälfte 10 und dichtet die Verbindungsbohrung 13a und die Nut 13b voneinander ab. Bei der Nut 13b handelt es sich bevorzugt um eine Umlaufnut.

Das Ausführungsbeispiel der Figur 12 zeichnet sich durch besonders gute äußere Abdichtungen bei den Dichtflächen 14a, 24a und 14b, 24b aus. In diesen Dichtbereichen sind die erste und die zweite Gehäusehälfte 10, 20 jeweils mit zueinander passenden Kegelmantelflächen 14a, 24a und 14b, 24b versehen, zwischen denen das stromaufwärtige und das stromabwärtige Ende des Ventilkörpers 37 beim Zusammenfügen der Gehäuse- hälften eingeklemmt wird. Über der Verbindungsbohrung 13a, dem Zwischensteg und der Nut 13b ist in der zweiten Gehäusehälfte 20 ausreichend Raum gelassen, in den sich der Ventilkörper 37 hinein ausdehnen kann, um die Strömungsverbindung zwischen der Verbindungsbohrung 13a und der Nut 13b herzustellen, wenn der Vorspanndruck überschritten worden ist. Mit gestrichelten Linien ist der aufgeweitete Zustand des Ventilkörpers 37 angedeutet. In der zweiten Gehäusehälfte 20 ist schließlich eine Druckausgleichsöffnung 29 gelassen, so dass am Ventilkörper 37 außenseitig stets Umgebungsdruck herrscht.

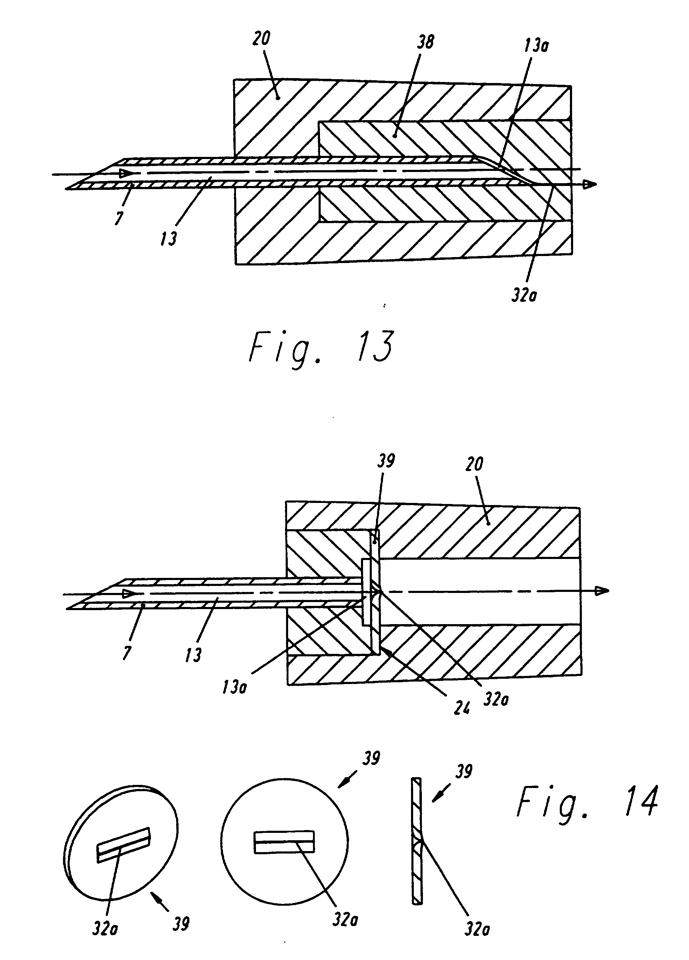

Figur 13 zeigt ein einteiliges Gehäuse 20, vergleichbar dem der Figuren 10 und 11, das in seinem stromaufwärtigen Bereich mit einer schmalen ersten und in seinem stromabwärtigen Bereich mit einer demgegenüber breiteren zweiten Bohrung versehen ist. Die erste Bohrung mündet in die zweite und dient als enge Führung und Sitz für die Verbindungsnadel 7. In die stromabwärtige breitere Bohrung ist ein Ventilkörper in Form eines einfachen Dichtpfropfens 38 eingepresst oder wieder eingegossen. Das Ventil wird beim Einführen der Verbindungsnadel 7 hergestellt, indem die Verbindungsnadel 7 vollkommen durch den Dichtpfropfen 38 durchgestochen und nach dem Durchstechen ein Stück weit wieder zurückgezogen wird. Auf diese Weise entsteht ein Durchlass 32a im Dichtpfropfen 38. Das Ventil der Figur 13 weist den Vorteil auf, dass die Verbindungsnadel 7 bzw. die Zuführleitung 13 durch einfaches Abschneiden aus einer Endloskanüle hergestellt werden kann.

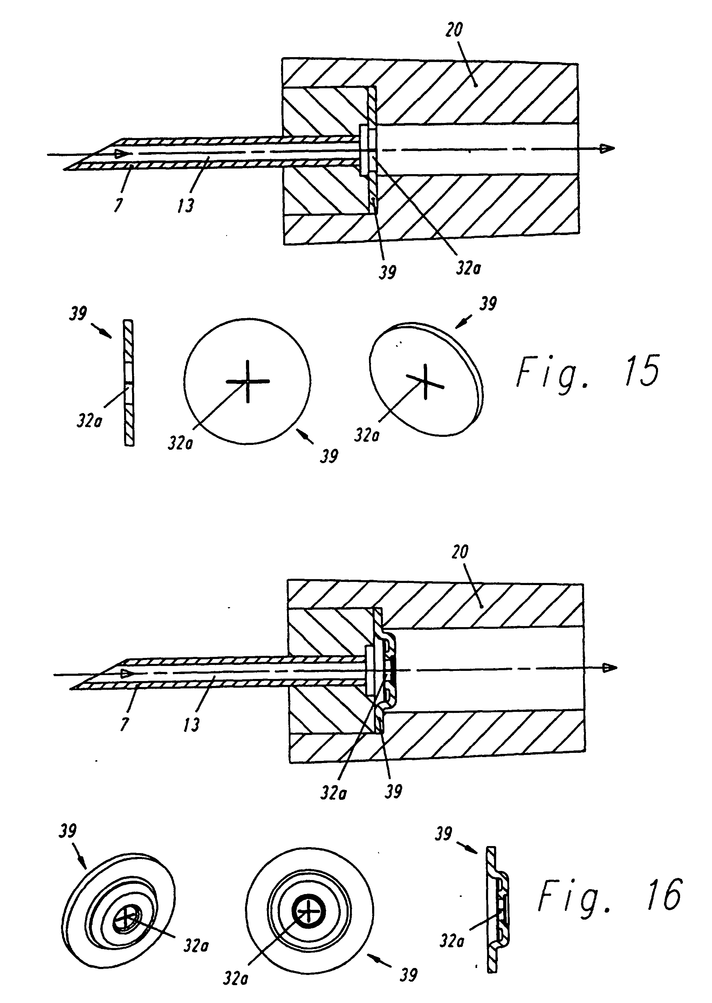

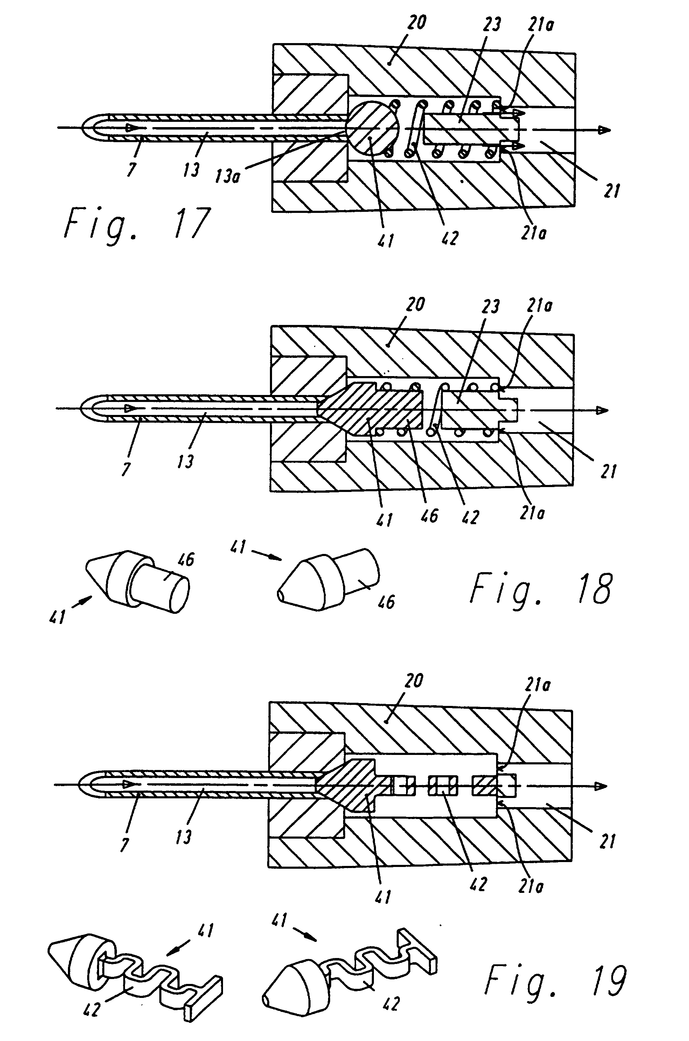

Die Ausführungsbeispiele nach den Figuren 14 bis 16 zeigen Ventile, die in der Art einer Herzklappe arbeiten. Die Ventilkörper 39 der Figuren 14 und 15 werden durch einfache Kreisscheiben gebildet, in die Durchlassschlitze 32a eingearbeitet sind. Die Gehäuse weisen jeweils ein in die zweite Gehäusehälfte 20 eingesetztes Montageeinsatzstück 10 auf mit je einer einfachen Einlassbohrung, die, gegebenenfalls wie in Figur 16 über eine Zwischenstufe, in die Auslassbohrung in der zweiten Gehäusehälfte 20 mündet. Die Ventilkörper 39 werden durch die stromabwärtige Stirnfläche des Montageeinsatzstücks 10 gegen die am Übergang zwischen Einlass- und Auslassbohrung umlaufende Schulter in der Gehäusehälfte 20 gepresst.

Während der Ventilkörper 39 der Figur 14 nur einen Schlitz mit in Strömungsrichtung ausgeformten Dichtlippen 32a aufweist, besitzt der Ventilkörper 39 der Figur 15 zwei sich kreuzende Schlitze 32a.

In der Anordnung nach Figur 16 ist unmittelbar stromabwärts von dem Ventilkörper 39 im Gehäuse 20 ein Hohlraum gelassen, in den sich der Ventilkörper 39 hinein dehnen kann. Auch dieser Ventilkörper 39 ist geschlitzt. Unter dem anstehenden Flüssigkeitsdruck füllt er sich wie eine Blase bis er schließlich öffnet. Er ist weniger starr als die vorbeschriebenen Ventilkörper 39 der Figuren 14 und 15.

In den Figuren 17 bis 21 sind Ventile dargestellt, deren elastische Ventilkörper 41 zur Erzielung der gewünschten Ventilwirkung von Druckfedern beaufschlagt werden.

Der Ventilkörper 41 des Ventils nach Figur 17 ist kugelförmig und wird durch eine Druckfeder 42 in Gegenströmrichtung in die als Ventilsitz dienende stromabwärtige Öffnung 13a der Zuführleitung 13 gepresst. Die Druckfeder 42 wird durch einen zentralen, auf die stromabwärtige Öffnung der Zuführleitung 13 zu weisenden Zylinder 23 des Gehäuses 20 geführt. An seinem stromabwärtigen Ende ist dieser Zylinder 23 mit einem Flansch mit Durchgangsöffnungen 21a versehen, durch die die Flüssigkeit nach Öffnen des Ventils in die Auslassleitung 21 strömt.

Figur 18 zeigt eine der Figur 17 vergleichbare Anordnung. Der Ventilkörper 41 der Figur 19 ist an seinem stromaufwärtigen Ende konisch verjüngt. Die stromabwärtige Öffnung der Zuführleitung 13 als Ventilsitz weitet sich entsprechend konisch auf. Desweiteren ist am stromabwärtigen Ende des Ventilkörpers 41 ein zylindrischer Fortsatz 46 ausgebildet, der der Führung der Druckfeder 42 am Ventilkörper 41 dient.

Der Ventilkörper 41 der Figur 19 drückt wieder mit einer kegelförmigen Oberfläche in den durch die Öffnung der Zuführleitung 13 gebildeten Ventilsitz. Die Andruckkraft wird durch eine Kunststofffeder 42 erzeugt.

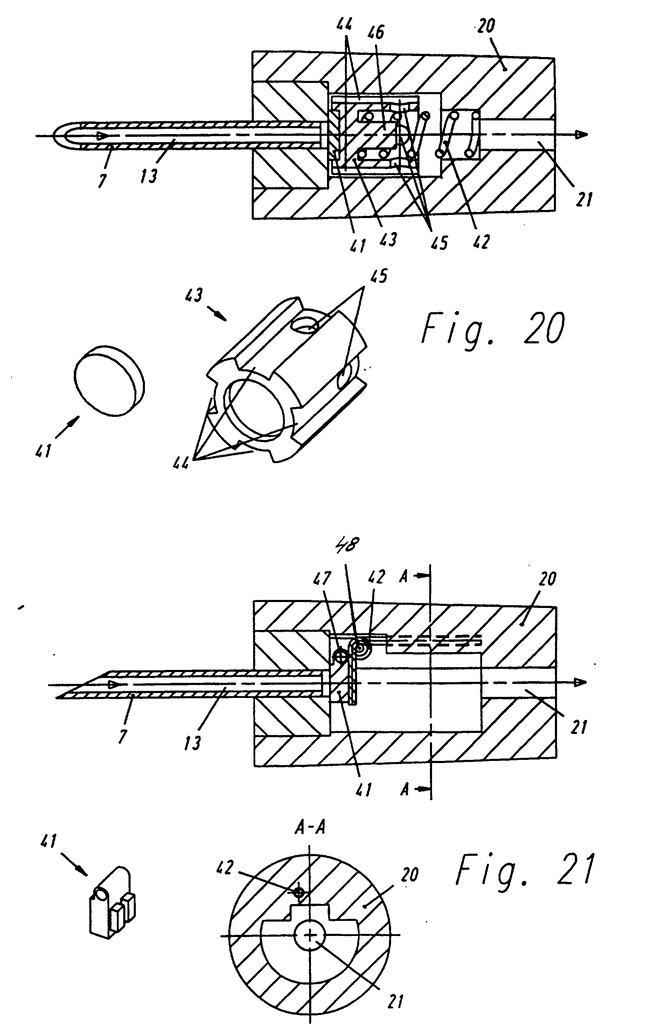

In Figur 20 ist der vorzugsweise elastische Ventilkörper 41 eine einfache Scheibe, die an der stromaufwärtigen Stirnfläche eines zylindrischen Führungskörpers 43 befestigt ist. Der Führungskörper 43 ist an seinem äußeren Mantel mit Längsnuten 44 versehen. Der Führungskörper 43 ist hohlzylindrisch mit einem Zylinderboden am stromaufwärtigen Ende, auf dem der Ventilkörper 41 fixiert aufliegt und von dem ein innerer Führungsfortsatz 46 in Strömungsrichtung abragt. Über diesen Führungsfortsatz 46 ist die Druckfeder 42 gezogen. Der Führungskörper 43 ist in seinen Längsnuten 44 mit radialen Durchgangsöffnungen 45 versehen, durch die die Medikamentflüssigkeit ins Innere des hohlzylindrischen Führungskörpers 43 und von dort durch die Auslassleitung 21 in den Katheter gelangen kann.

Beim Ventil der Figur 21 ist der Ventilkörper 41 als Rückschlagklappe um eine Drehachse 47 quer zur Strömungsrichtung unmittelbar an der stromabwärtigen Öffnung der Zuführleitung 13 angeordnet. An ihrer stromabwärtigen Rückseite wird diese Rückschlagklappe 41 durch eine Schenkelfeder 42 zum Schließen der Zuführleitungsöffnung mit Druck beaufschlagt. Die Schenkelfeder 42 ist so in die Gehäusehälfte 20 eingelegt, dass ihre Federachse 48 parallel zur Rückschlagklappen-Drehachse 47 ist, womit der erste Schenkel der L-förmigen Feder 42 gegen die Rückseite der Rückschlagklappe 41 und der zweite Schenkel gegen die als Gegenlager dienende innere Wandung des Gehäuses 20 drücken. Durch die Aufspreizkraft der abgewinkelten Schenkelfeder 42 wird die Rückschlagklappe 41 dicht gegen die stromabwärtige Öffnung der Zuführleitung 13 gepresst. Der zweite Federschenkel ragt in eine axiale Bohrung in der zweiten Gehäusehälfte 20, und der erste Schenkel ist in einer Nut an der Rückseite des Ventilkörpers 41 geführt; er könnte auf fest mit dem Ventilkörper 41 verbunden sein. Die Federachse 48 wird allein durch die Schenkelfeder 42 gebildet, eine weitere Lagerstelle ist bei 48 nicht notwendig.

Die jeweils in konkreter Kombination miteinander beschriebenen Ventilkörper und Gehäuse können auch jeweils mit in den anderen Kombinationen beschriebenen Gehäusen und Ventilkörpern verwendet werden.

## Patentansprüche

1. Vorrichtung zur dosierten Verabreichung, insbesondere Infusion, einer Medikamentenflüssigkeit, mit
a) einem Gehäuse (G),
b) einem von dem Gehäuse (G) aufgenommenem Behältnis (1), aus dem die Medikamentenflüssigkeit zu ihrer Verabreichung dosiert durch einen Auslass (4) verdrängt wird, und
c) einem Anschlussgehäuse (20), das den Auslass (4) mit einem Katheter (8) verbindet, dessen vom Anschlussgehäuse (20) abgewandtes Ende mit einer Verabreichungsnadel (9) verbunden oder verbindbar ist, und
d) einem von dem Anschlussgehäuse (20) gelagerten Ventil (30), das in einem Strömungsquerschnitt der Medikamentenflüssigkeit angeordnet ist und zum Verhindern einer Selbstentleerung einen Durchfluss auf das vordere Ende des Katheters (8) zu nur zulässt, wenn der in diese Richtung wirkende Flüssigkeitsdruck größer ist als ein auf dem Ventil (30) lastender Druck infolge des Eigengewichts einer Flüssigkeitssäule in der Vorrichtung,
**dadurch gekennzeichnet, dass**
e) die Medikamentenflüssigkeit durch Vorschub eines Stopfens (2) durch den Auslass (4) verdrängt wird,
f) und das Anschlussgehäuse (20) lösbar an dem Auslass (4) angeschlossen ist und eine Verbindungsnadel (7) so lagert, dass die Verbindungsnadel (7) eine den Auslass (4) verschließende Membran durchsticht, wenn das Anschlussgehäuse (20) angeschlossen wird.

2. Vorrichtung nach Anspruch 1, **dadurch gekennzeichnet, dass** das Ventil (30) ein passives Einwegventil ist.

3. Vorrichtung nach Anspruch 1 oder 2, **dadurch gekennzeichnet, dass** das Ventil (30) den Durchfluss erst zulässt, wenn der Flüssigkeitsdruck den maximal möglichen Druck der Flüssigkeitssäule übersteigt.

4. Vorrichtung nach dem vorhergehenden Anspruch, **dadurch gekennzeichnet, dass** das Ventil (30) den Durchfluss erst zulässt, wenn der Flüssigkeitsdruck den maximal möglichen Druck der Flüssigkeitssäule multipliziert mit einem Sicherheitsfaktor größer 1, insbesondere ein Sicherheitsfaktor im Bereich von 2 bis 4, übersteigt.

5. Vorrichtung nach einem der vorhergehenden Ansprüche, **dadurch gekennzeichnet, dass** das Ventil (30) einen Ventilkörper (31; 37; 38; 39; 41) aufweist, der gegen wenigstens eine Öffnung (13a) einer zum Ventilkörper (31; 37; 38; 39; 41) führenden Zuführleitung (13) für die Medikamentflüssigkeit vorgespannt ist, wobei die Größe der Vorspannkraft so gewählt ist, dass sie an einer die Öffnung (13a) dichtend umschließenden Kontaktfläche (33) des Ventilkörpers (31; 37; 38; 39; 41) eine Kraft erzeugt, die größer ist, als die durch die Flüssigkeitssäule auf den beaufschlagten Ventilquerschnitt ausgeübte Kraft.

6. Vorrichtung nach dem vorhergehenden Anspruch, **dadurch gekennzeichnet, dass** die Kontaktfläche (33) an einer die Öffnung (13a) umschließenden Dichtlippe (15; 35) gebildet wird.

7. Vorrichtung nach dem vorhergehenden Anspruch, **dadurch gekennzeichnet, dass** der Ventilkörper (31) über der Dichtlippe (15; 35) in Richtung auf eine stromaufwärts von der Dichtlippe (15; 35) gelegene Wandung (14) eines den Ventilkörper (31) aufnehmenden flüssigkeitsdichten Gehäuses (10, 20; 20) gespannt ist.

8. Vorrichtung nach Anspruch 6 oder 7, **dadurch gekennzeichnet, dass** die Dichtlippe (15) an der Zuführleitung (13) ausgebildet ist.

9. Vorrichtung nach Anspruch 6, **dadurch gekennzeichnet, dass** die Dichtlippe (35) quer zur Strömungsrichtung gegen eine den Strömungsquerschnitt umschließende Umfangsfläche (19; 24) drückt.

10. Vorrichtung nach Anspruch 5, **dadurch gekennzeichnet, dass** der Ventilkörper (37) eine Mantelfläche der Zuführleitung (13) umschließt und die Zuführleitung (13) in ihrem von dem Ventilkörper (37) umschlossenen Bereich mit der wenigstens einen den Strömungsquerschnitt bildenden Öffnung (13a) versehen ist.

11. Vorrichtung nach Anspruch 5 oder 10, **dadurch gekennzeichnet, dass** der Ventilkörper (37; 38) ein Dichtpfropfen ist, der die in ihn hineinragende Zuführleitung (13) dichtend umschließt und zur Ausbildung eines sich an die wenigstens eine Öffnung (13a) der Zuführleitung (13) anschließenden Strömungsquerschnitts aufweitbar ist.

12. Vorrichtung nach Anspruch 5, **dadurch gekennzeichnet, dass** der Ventilkörper (39) den Strömungsquerschnitt in der Art einer Herzklappe verschließt und freigibt.

13. Vorrichtung nach Anspruch 5, **dadurch gekennzeichnet, dass** der Ventilkörper (41) durch Andruck einer Feder (42) gegen die wenigstens eine Öffnung (13a) der Zuführleitung (13) gedrückt wird.

14. Vorrichtung nach einem der vorhergehenden Ansprüche, **dadurch gekennzeichnet, dass** das Anschlussgehäuse (20) an einem den Auslass (4) verlängernden Auslassstutzen (5) des Behältnisses (1) befestigt ist.

## Claims

1. A device for administering, in particular infusing, a medicine fluid in doses, comprising:
a) a casing (G);
b) a container (1) which is accommodated by the casing (G) and from which the medicine fluid is expelled through an outlet (4) in order to administer it in doses;
c) a connector casing (20) which connects the outlet (4) to a catheter (8), wherein the end of the catheter (8) facing away from the connector casing (20) is or can be connected to an administering needle (9); and
d) a valve (30) which is mounted by the connector casing (20), is arranged in a flow cross-section of the medicine fluid, and in order to prevent the device from emptying by itself, only allows a flow towards the front end of the catheter (8) when the fluid pressure acting in this direction is greater than a pressure resting on the valve (30) as a result of the dead weight of a fluid column in the device;
**characterised in that**:
e) the medicine fluid is expelled through the outlet (4) by advancing a stopper (2);
f) and the connector casing (20) is detachably connected to the outlet (4) and mounts a connecting needle (7) such that the connecting needle (7) penetrates a membrane sealing the outlet (4) when the connector casing (20) is connected.

2. The device as set forth in claim 1, **characterised in that** the valve (30) is a passive unidirectional valve.

3. The device as set forth in claim 1 or 2, **characterised in that** the valve (30) does not allow the flow until the fluid pressure exceeds the maximum possible pressure of the fluid column.

4. The device as set forth in the preceding claim, **characterised in that** the valve (30) does not allow the flow until the fluid pressure exceeds the maximum possible pressure of the fluid column, multiplied by a safety factor greater than 1, in particular a safety factor in the range from 2 to 4.

5. The device as set forth in any one of the preceding claims, **characterised in that** the valve (30) comprises a valve body (31; 37; 38; 39; 41) which is biased against at least one opening (13a) of a supply line (13) for the medicine fluid which leads to the valve body (31; 37; 38; 39; 41), wherein the magnitude of the biasing force is selected such that it generates a force on a contact area (33) of the valve body (31; 37; 38; 39; 41) which encompasses the opening (13a), forming a seal, said force being greater than the force exerted on the charged valve cross-section by the fluid column.

6. The device as set forth in the preceding claim, **characterised in that** the contact area (33) is formed on a sealing lip (15; 35) encompassing the opening (13a).

7. The device as set forth in the preceding claim, **characterised in that** the valve body (31) is tensed above the sealing lip (15; 35) towards a wall, upstream of the sealing lip (15; 35), of a fluid-tight casing (10, 20; 20) accommodating the valve body (31).

8. The device as set forth in claim 6 or 7, **characterised in that** the sealing lip (15) is formed on the supply line (13).

9. The device as set forth in claim 6, **characterised in that** the sealing lip (35) presses transverse to the flow direction against a circumferential area (19; 24) encompassing the flow cross-section.

10. The device as set forth in claim 5, **characterised in that** the valve body (37) encompasses a surface area of the supply line (13) and the region of the supply line (13) encompassed by the valve body (37) is provided with the at least one opening (13a) forming the flow cross-section.

11. The device as set forth in claim 5 or 10, **characterised in that** the valve body (37; 38) is a sealing plug which encompasses the supply line (13) protruding into it, forming a seal, and can be expanded in order to form a flow cross-section connected to the at least one opening (13a) of the supply line (13).

12. The device as set forth in claim 5, **characterised in that** the valve body (39) seals and exposes the flow cross-section in the manner of a cardiac valve.

13. The device as set forth in claim 5, **characterised in that** the valve body (41) is pressed by pressing a spring (42) against the at least one opening (13a) of the supply line (13).

14. The device as set forth in any one of the preceding claims, **characterised in that** the connector casing (20) is fastened to an outlet support (5) of the container (1) which lengthens the outlet (4).

## Revendications

1. Dispositif pour l'application dosée, en particulier par perfusion, d'un médicament liquide, avec
a) un boîtier (G),
b) un contenant (1) logé par le boîtier (G), hors duquel le médicament liquide pour son application dosée est expulsé à travers une sortie (4), et
c) un boîtier de raccordement (20), qui relie la sortie (4) à un cathéter (8) dont l'extrémité opposée au boîtier de raccordement (20) est reliée ou peut être reliée à une aiguille d'application (9), et
d) une vanne (30) logée par le boîtier de raccordement (20), qui est disposée dans une section transversale de circulation du médicament liquide et qui, pour empêcher un auto vidage, ne permet un passage vers l'extrémité avant du cathéter (8) que si la pression du liquide s'exerçant dans cette direction est supérieure à une pression s'exerçant sur la vanne (30) à la suite du poids propre d'une colonne de liquide dans le dispositif,
**caractérisé en ce que**
e) le médicament liquide est expulsé par avance d'un bouchon (2) à travers la sortie (4),
f) et **en ce que** le boîtier de raccordement (20) est raccordé de manière amovible à la sortie (4) et loge une aiguille de liaison (7) de manière à ce que l'aiguille de liaison (7) transperce une membrane fermant la sortie (4), lorsque le boîtier de raccordement (20) est raccordé.

2. Dispositif selon la revendication 1, **caractérisé en ce que** la vanne (30) est une vanne passive à voie unique.

3. Dispositif selon la revendication 1 ou 2, **caractérisé en ce que** la vanne (30) ne permet le passage que si la pression du liquide dépasse la pression maximale possible de la colonne de liquide.

4. Dispositif selon la revendication précédente, **caractérisé en ce que** la vanne (30) ne permet le passage que si la pression du liquide dépasse la pression possible maximale de la colonne de liquide multipliée par un facteur de sécurité supérieur à 1, en particulier un facteur de sécurité situé dans un domaine de 2 à 4.

5. Dispositif selon l'une quelconque des revendications précédentes, **caractérisé en ce que** la vanne (30) comporte un corps de vanne (31 ; 37 ; 38 ; 39 ; 41), qui est prétendu contre au moins une ouverture (13a) d'un tube d'alimentation (13) pour le médicament liquide menant vers le corps de vanne (31 ; 37 ; 38 ; 39 ; 41), sachant que la grandeur de la force de pré-tension est choisie de telle sorte qu'elle produise une force sur une surface de contact (33) du corps de vanne (31 ; 37 ; 38 ; 39 ; 41) enfermant de manière étanche l'ouverture (13a), force qui est supérieure à celle exercée par la colonne de liquide sur la section transversale de vanne sollicitée.

6. Dispositif selon la revendication précédente, **caractérisé en ce que** la surface de contact (33) est formée à une lèvre d'étanchéité (15 ; 35) enfermant l'ouverture (13a).

7. Dispositif selon la revendication précédente, **caractérisé en ce que** le corps de vanne (31) est tendu au-dessus de la lèvre d'étanchéité (15 ; 35) en direction d'une paroi (14) d'un boîtier (10 ; 20 ; 20) logeant de manière étanche au liquide le corps de vanne (31), placée en amont par rapport au flux de la lèvre d'étanchéité (15 ; 35).

8. Dispositif selon la revendication 6 ou 7, **caractérisé en ce que** la lèvre d'étanchéité (15) est conçue sur le tube d'alimentation (13).

9. Dispositif selon la revendication 6, **caractérisé en ce que** la lèvre d'étanchéité (35) pousse en oblique par rapport à la direction de circulation contre une surface périphérique (19 ; 24) enfermant la section transversale de circulation.

10. Dispositif selon la revendication 5, **caractérisé en ce que** le corps de vanne (37) enferme une surface d'enveloppe du tube d'alimentation (13) et **en ce que** le tube d'alimentation (13) est muni dans sa zone enfermée par le corps de vanne (37) de l'ouverture (13a) formant au moins une des sections transversales de circulation.

11. Dispositif selon la revendication 5 ou 10, **caractérisé en ce que** le corps de vanne (37 ; 38) est un bouchon étanche qui enferme de manière étanche le tube d'alimentation (13) dépassant dans celui-ci et qui peut être étendu pour la formation d'une section transversale de circulation se raccordant au moins à une ouverture (13a) du tube d'alimentation (13).

12. Dispositif selon la revendication 5, **caractérisé en ce que** le corps de vanne (39) ferme et libère la section transversale de circulation à la façon d'une valvule.

13. Dispositif selon la revendication 5, **caractérisé en ce que** le corps de vanne (41) est poussé par pression d'un ressort (42) contre au moins une ouverture (13a) du tube d'alimentation (13).

14. Dispositif selon l'une quelconque des revendications précédentes, **caractérisé en ce que** le boîtier de raccordement (20) est fixé à un appui de sortie (5) du contenant (1) rallongeant la sortie (4).
